# EUROPÄISCHE PATENTANMELDUNG

(11) **EP 3 995 114 A1**
(43) Veröffentlichungstag der Anmeldung: **11.05.2022**
(21) Anmeldenummer: 20020513.6
(22) Anmeldetag: 08.11.2020
(51) Int. Cl.: A61F 2/78

(54) **PROTHESENLINER**

(71) Anmelder: Rockstroh, Martin, 95473 Creussen (DE)
(72) Erfinder: Rockstroh, Martin, 95473 Creussen (DE)

(57) **Zusammenfassung**

Prothesenliner (1) zum Überziehen über einen Gliedmaßenstumpf zur Fixierung eines Prothesenschafts (6), bestehend aus einen über den Stumpf zu ziehenden Grundkörper (2, 2', 2") sowie zumindest einen mit dem Grundkörper (2, 2', 2") verbundenen ersten Gestrickabschnitt (3, 3', 3"), wobei in den zumindest einen ersten Gestrickabschnitt (3, 3', 3") zumindest abschnittsweise ein Haftfaden (4) eingestrickt ist, der vorzugsweise adhäsiv mit dem Gliedmaßenstumpf und/ oder dem Prothesenschaft (6) zusammenwirkt.

## Beschreibung

Die vorliegende Erfindung betrifft einen Prothesenliner zum Überziehen über einen Gliedmaßenstumpf zur Fixierung eines Prothesenschafts nach dem Oberbegriff des Anspruch 1 sowie auf eine Prothesenanordnung mit einem derartigen Prothesenliner sowie einen ein Ventil aufweisenden Prothesenschaft nach dem Oberbegriff des Anspruch 15.

Zur Fixierung eines Prothesenschaftes beispielsweise einer Unterschenkel- oder Oberschenkelprothese kommen zunehmend sogenannte Prothesenliner zum Einsatz. Die Liner bestehen aus einem flexiblen Grundkörper, welcher insbesondere aus einem Silikon gefertigt ist, so dass er sich gut der Form des Gliedmaßenstumpfes, über den er gezogen wird, anpassen kann. Im Bereich des distalen Endes des Linerkörpers ist wenigstens eine, zumeist mehrere axial voneinander beabstandete Dichtelemente vorgesehen. Diese Dichtelemete legen sich an die Innenseite des Prothesenschafts an, wenn der mit dem Liner versehene Stumpf in den Prothesenschaft eingeführt wird. Durch das Einführen wird Luft aus dem Prothesenschaft herausgedrückt, wozu üblicherweise ein entsprechendes Ventil vorgesehen ist. Sollte der Stumpf mit Liner aus dem Prothesenschaft herausgezogen werden, entsteht ein Unterdruck im Inneren des Prothesenschafts, welcher das Herausziehen verhindert. Um die Verschleißfestigkeit weiter zu verbessern ist, an bzw. in dem Liner oftmals ein textiles Verstärkungsmaterial vorgesehen. Zudem kann der Liner mittels eines Pins fest mit dem Schaft verbunden werden. Hierbei wird der Pin in einer schaftseitige Verriegelung fest gehalten.

Der Prothesenschaft ist in seiner Geometrie bzw. dem umschlossenen Volumen im Wesentlichen dem Volumen des Stumpfes mit angelegtem Prothesenliner angepasst, so dass der Stumpf hinreichend fest und spielfrei im Schaft aufgenommen ist. Es kommt jedoch zu Schwankungen des Stumpfvolumens, das heißt, dass der Stumpf umfangsmäßig etwas abnimmt, so dass ein fester Sitz im Schaft nicht mehr unbedingt gegeben ist. Es ist bekannt, über den Prothesenliner einen Gestricküberzug zur Kompensation zu ziehen, der den Gesamtumfang im Stumpfbereich wieder etwas vergrößert, so dass die Volumenabnahme hierüber ausgeglichen und der Stumpf wieder hinreichend stabil im Schaft aufgenommen ist.

Zur Verbesserung des Halts des Stumpfes in dem Prothesenschaft kann zwischen Prothesenliner und Prothesenschaft, alternativ zu einem Gestricküberzug auch ein externes Dichtelemente, insbesondere in Form eines Dichtrings, vorgesehen sein. Dieser wird auf der vorzugsweise textilen Außenseite des Prothesenliners befestigt. Damit nun keine Luft zwischen dem Textil und dem Dichtring entkommen kann, weist das Textil oder der Schaft zusätzliche Dichtelemente auf.

Aus dem Stand der Technik sind eine Vielzahl von Porthesenliner zum Überziehen über einen Gliedmaßenstumpf zur Fixierung eines Prothesenschafts bekannt.

Ein derartiger Prothesenliner, insbesondere ausgebildet als Silikonliner, wird beispielsweise in der EP 2815728 B1 offenbart.

Dieser bekannte Prothesenliner weist einen Grundkörper aus Silikon auf, an welchem ein über diesen außenseitig zu ziehenden Gestricküberzug zur Kompensation von Stumpfvolumenschwankungen, befestigt ist. Der Gestricküberzug ist lediglich im Bereich des distalen Endes angeordnet. Zum besseren Halt des Gestricküberzugs am Liner, weist diese an der Innenseite, also der dem Liner zugewandten Seite, eine Haftschicht auf. Diese kann in Form eines Haftbandes aufgenäht oder aufgeklebt sein. Alternativ kann die Haftschicht auch auf das Gestrick aufgegossen, aufgestrichen oder auf das Gestrick aufgeschmolzen sein. Ferner weist der Liner wenigstens eine im Bereich des distalen Endes ringförmig umlaufende Dichtlippe zur dichten Anlage an der Innenseite des Prothesenschafts auf. Die Dichtlippen sind einteilig mit dem Grundkörper aus Silikon gefertigt.

Auch die EP3238667 A1 offenbart einen derartigen Prothesenliner aus Silikon. Der Liner weist einen hohlen Grundkörper aus Silikon auf, wobei wenigstens eine umlaufenden, seitlich vom Linerkörper abstehende flexiblen Dichtlippe zur dichten Anlage an der Innenseite des Prothesenschafts vorgesehen ist. Die Dichtlippe ist ebenfalls aus einem flexiblen Material, vorzugsweise dem gleichen Material wie dem des Linerkörper, also einem Silikon, gefertigt. Die Höhe der Dichtlippe variiert um ihren Umfang von einem ersten Höhenwert an einer ersten Umfangsposition oder in einem ersten Umfangsbereich zu einem niedrigeren zweiten Höhenwert an einer zweiten Umfangsposition oder in einem zweiten Umfangsbereich.

Aus der EP 3298991 A1 ist ein Dichtungssystem für einen Prothesenliner bekannt. Das einstellbare Dichtungssystem zum Bereitstellen einer Schnittstelle zwischen einem Restglied und einem Prothesenschaft umfasst einen Liner mit einem Linerkörper sowie einer Dichtungskomponente in Form eines Dichtrings. Dieser umfasst einen Grundkörper, der aus einem Polymermaterial gefertigt ist, mehrere an der Außenseite angeordnete Dichtlippen zum Abdichten gegenüber einen Prothesenschaft, sowie eine an der Außenseite angebrachte Textilhülse. Der Prothesenliner selbst weist an dessen Außenseite bevorzugt ebenfalls ein Textil auf, an welchem der Dichtring befestigt wird. Zur Abdichtung zwischen Prothesenliner und Dichtring sind an dem Textil mehrere Dichtringe vorgesehen. Diese, insbesondere geeignetes Material, wird hierzu auf das Textil aufgetragen, insbesondere aufgeschmolzen.

Als nachteilig dieser aus dem Stand der Technik bekannten und allgemein üblichen Ausbildung von Prothesenliner, insbesondere die Abdichtung des Liners gegenüber dem Schaft zur Erzeugung eines Vakuums im unteren Bereich des Prothesenschaft, um ein Herausrutschen des Gliedmaßenstumpfs zu vermeiden, sowie den Kontakt und Halt des Prothesenliners in dem Schaft selbst zu gewährleisten, hat sich die aus dem Stand der Technik bekannte und übliche Aufbringung von Dichtelementen erwiesen.

Das zur Verfügung stellen und Aufbringen der separaten Dichtelemente durch auftragen bzw. aufschmelzen eines geeigneten Materials auf das den Liner umgebende Gestrick führ im Rahmen der Fertigung des Prothesenliners zu einem zusätzlichen Prozessschritt, was somit die Produktionskosten des Liners erhöht.

Ein weiterer Nachteil der damit verbunden ist, ist die mangelnde Haltbarkeit der Dichtelemente an dem Gestrick. Diese werden lediglich oberflächig mit dem Gestrick verbunden. Durch Reibung zwischen dem Prothesenliner und Prothesenschaft sowie durch das Vielfache Ein- und Aussteigen in bzw. aus dem Prothesenliner, kommt es zu einer sehr hohen Beanspruchung der Dichtelemente. Dies führt folglich dazu, dass sich die Dichtelemente vom dem Gestrick lösen. Eine sichere Abdichtung zwischen Liner und Prothesenschaft kann somit nicht auf Dauer gewährleistet werden.

Schließlich erweist sich auch als nachteilig, dass die Dichtelemente nur einseitig an dem Liner aufgebracht sind. Diese bekannten Dichtelemente ermöglichen somit nur eine Abdichtung zwischen Prothesenliner und Schaft. Eine gleichzeitige Abdichtung, auf Grund von Volumenschwankungen, zwischen Gliedmaßenstumpf und Prothesenliner wird nicht ermöglicht. Auch eine Abdichtung innerhalb des Gestricks bzw. Textils, auf den die Dichtelemente aufgebracht sind, kann nicht mit Sicherheit gewährleistet werden, da das Aufschmelzen des Materials auf das Gestrick mit zu hohen Temperaturen das Gestrick beschädigen würden. Somit erstrecken sich die Dichtelemente oftmals, wenn überhaupt, nur teilweise in das Gestrickinnere.

Ferner bringen die durch Aufschmelzen und Aufspritzen hergestellten Dichtelemente den Nachteil mit sich, dass hierdurch nur ein dicker und ungenauer Aufbau der Dichtelemente möglich ist. Minimale bzw. punktgenau Dichtelemente und Erhebungen sind damit gar nicht möglich.

Auch hat sich als nachteilig erwiesen, dass die aus dem Stand der Technik bekannten Methoden zur Fertigung des Prothesenliners, insbesondere des Grundkörpers selbst, mit enormem Aufwand und einer Vielzahl von Fertigungsschritten verbunden ist. Bspw. müssen von einem Gliedmaßenstumpf ein Gipsabdruck erstellt werde, von dem wiederum der aus Silikon gegossene Grundkörper gefertigt wird. Diese Methode zur Fertigung der Prothesenliner hat sich zwar am Markt durchgesetzt, jedoch ist diese Methode aufwendig und kostspielig.

Einen weiteren Nachteil, welchen die bekannten Methoden zur Herstellung solcher Prothesenliner, insbesondere des Grundkörpers mit sich bringen ist, dass die bekannten Prothesenliner einen sehr dicken Wandaufbau mit sich bringen. Folglich kommt es zu klobigen Ausführungen der Liner, welche ein nicht unerhebliches Eigengewicht mit sich bringen.

Der vorliegenden Erfindung liegt daher die Aufgabe zugrunde einen Prothesenliner zu schaffen, welcher die Nachteile aus dem Stand der Technik vermeidet, insbesondere die Abdichtung und somit die Fixierung des Liners zwischen bzw. gegenüber dem Gliedmaßenstumpf und dem Prothesenschaft deutlich verbessert. Ebenfalls ist es Aufgabe die Fertigung eines derartigen Liners wesentlich zu vereinfachen.

Gemäß einem Ausführungsbeispiel des Prothesenliners zum Überziehen über einen Gliedmaßenstumpf zur Fixierung eines Prothesenschafts besteht dieser aus einen über den Stumpf zu ziehenden Grundkörper sowie zumindest einen mit dem Grundkörper verbundenen ersten Gestrickabschnitt, wobei in den zumindest einen ersten Gestrickabschnitt zumindest abschnittsweise ein Haftfaden eingestrickt ist, der vorzugsweise adhäsiv mit dem Gliedmaßenstumpf und/ oder dem Prothesenschaft zusammenwirkt.

Gemäß einem zweiten Ausführungsbeispiel ist der zumindest eine erste Gestrickabschnitt lösbar mit dem Grundkörper verbunden. Hierdurch ist es möglich den ersten Gestrickabschnitt austauschbar auszubilden. Bspw. können Gestricke bzw. Überzüge für den Prothesenliner mit unterschiedlich hohen Dichtelemente zur Verfügung gestellt werden.

Gemäß einem dritten Ausführungsbeispiel ist der zumindest eine erste Gestrickabschnitt unlösbar mit dem Grundkörper verbunden. Hierbei ist bevorzugt der Grundkörper durch einen zweiten Gestrickabschnitt, insbesondere einem Abstandsgestrick, gebildet. Dabei handelt es sich dann um einen vollständig gestrickten Prothesenliner. Das Abstandgestrick wird hierbei durch sogenannte Bauschfäden gestrickt. Die Stärke des Gestricks, also die Dicke des Grundkörpers ist hierbei maschengenau ausbildbar.

Gemäß einem vierten Ausführungsbeispiel weist der erste und/ oder zweite vorzugsweise rundgestrickte Gestrickabschnitt zumindest einen Kompressionsbereich auf. Die Kompressionsstärke beträgt hierbei, gemessen mit dem Messystem HOSY der Fa. Hohenstein, bereichsweise vorzugsweise zwischen 10 und 50 mmHg.

Gemäß einem fünften Ausführungsbeispiel variiert die Wandstärke des Grundkörpers, insbesondere die des Abstandgestricks, in Umfangsrichtung und/ oder Längsrichtung. Hierdurch kann der Prothesenliner maschengenau, also punktgenau, oder maschenreihengenau dem Gliedmaßenstumpf angepasst werden.

Gemäß einem sechsten Ausführungsbeispiel ist der erste und/ oder zweite Gestrickabschnitt zur Bildung eines oder mehrerer Haft- und/ oder Dichtelemente derart ausgebildet, dass dieser an der Außen- und/ oder Innenseite des Prothesenliners zumindest abschnittsweise hervortritt. Bevorzugt weist der Gestrickabschnitt im Bereich der gebildeten Haft- und/ oder Dichtelemente eine ringförmige Querschnittsform auf. Hierdurch sind die Haft- und/ oder Dichtelemente vorzugsweise kreisförmig bzw. wulstförmig gestrickt.

Gemäß einem weiteren Ausführungsbeispiel ist der Haftfaden zur Bildung eines oder mehrerer Haft- und/ oder Dichtelemente derart in den ersten und/ oder zweiten Gestrickabschnitt eingestrickt ist, dass dieser an der Außen- und/ oder Innenseite des Prothesenliners zumindest abschnittsweise hervortritt. Der Faden ist hierbei bevorzugt auf einen Grundfaden aufplattiert, oder alleine mit einer zum Grundstrickfaden unterschiedlichen Maschengröße gestickt. Auch kann dieser mit einer zum Grundstrickfaden unterschiedlichen Vorspannung eingestrickt sein. Im Ergebnis ragen zumindest Abschnitte des verstrickten Haftfadens über das übrige Gestrick hinaus.

Gemäß einem weiteren Ausführungsbeispiel weist das eine oder die mehreren gebildeten Haft- und/ oder Dichtelemente jeweils eine Ausdehnung in Gestricklängs- und/ oder Umfangsrichtung zwischen 0,1 und 1 cm, maximal 2 cm, auf.

Gemäß einem weiteren Ausführungsbeispiel sind die mehreren Haft- und/ oder Dichtelemente unterschiedlich hoch. Bevorzugt beträgt die Höhe eines ersten gestrickten Haft- und/ oder Dichtelements zwischen 4 - 8 mm, insbesondere 6 mm, und die Höhe eines zweiten Haft- und/ oder Dichtelements zwischen 2 - 5 mm, insbesondere 4 mm.

Gemäß einem weiteren Ausführungsbeispiel variiert die Höhe des einen, der mehreren oder aller Haft- und/ oder Dichtelemente.

Gemäß einem weiteren Ausführungsbeispiel ist der Haftfaden ein Silikon-, Elastan- oder Kautschukfaden. Der Haftfaden weist bevorzugt einen Fadenkern, insbesondere aus Elastan, auf, wobei der Fadenkern mit Silikon, Elastan und/ oder Kautschuk beschichtet oder umwunden ist. Der Grundstrickfaden ist vorzugsweise aus Polyamid, Polyester, Polypropylen oder Naturfaser.

Gemäß einem Ausführungsbeispiel einer Prothesenanordnung besteht diese aus einem Prothesenliner gemäß einem der vorhergehenden Ausführungsbeispiele sowie einen ein Ventil aufweisenden Prothesenschaft.

Gemäß einem Ausführungsbeispiel zur Herstellung des Prothesenliners, insbesondere gemäß eines computer-implementieren Verfahrens, weist dieses die Schritte auf:
1) manuelles oder automatisches Erfassen, vorzugsweise mittels einer 3D Körperscaneinrichtung, die Maße eines Gliedmaßenstumpfes und Generierung eines Datensatzes mit den Körpermaßen des Gliedmaßenstumpfes daraus,
2) Auswerten des erfassten Datensatzes und Umrechnung der erfassten Körpermaße in Strickmaße und Ergänzen des bestehenden personenbezogenen Datensatzes um die Strickmaße oder Generierung eines neuen personenbezogenen Datensatzes beinhaltend die Strickmaße,
3) Stricken des Prothesenliners gemäß der vorgehenden Ausführungsbeispiele unter Verwendung des generierten Datensatzes beinhaltend die personenbezogenen Strickmaße;

Das vorliegende Beinbekleidungsstück zeichnet sich durch eine Reihe erheblicher Vorteile aus.

Durch die Ausbildung des Prothesenliners mit einem Grundkörper sowie einen damit verbundenen Gestrickabschnitt, wobei in den zumindest einen ersten Gestrickabschnitt zumindest abschnittsweise ein Haftfaden eingestrickt ist, der vorzugsweise adhäsiv mit dem Gliedmaßenstumpf und/ oder dem Prothesenschaft zusammenwirkt, ist ein maschengenaues Einstricken bzw. Erstellen von Haft- und/ oder Dichtbereichen möglich. Sowohl die Breite, die Länge als auch die Höhe der Dichtelemente ist exakt bestimmbar und ausführbar.

Durch das Einarbeiten der Haft- und/ oder Dichtbereiche im Rahmen der Herstellung des an dem Prothesenliner angeordneten Gestricks, also im selben Prozessschritt, wird ein weitere Fertigungsschritt vermieden. Hierdurch können Fertigungskosten reduziert werden. Dies insbesondere dann, wenn auch der Grundkörper aus einem zweiten Gestrickabschnitt, insbesondere einem Abstandsgestrick, gebildet ist. Dadurch ist es im Wesentlichen möglich den kompletten Prothesenliner durch einen einzigen Fertigungsschritt, nämlich Strickprozess, herzustellen.

Ein weiterer Vorteil der Erfindung ist, dass durch die stricktechnische Ausbildung des Grundkörpers eine besonders leichte und exakte Ausbildung des Grundkörpers möglich ist. Dies bringt wiederum Gewichtsersparnis mit sich, was den Tragekomfort des Liners wesentlich verbessert.

Schließlich hat die stricktechnische Ausbildung der Haft- und/ oder Dichtbereiche den Vorteil, dass diese an beiden Seiten des Gestricks bzw. des Grundkörpers ausgebildet werden können. Diese können sich hierbei sogar durch das Gestrick erstrecken, was dazu führt, dass ein besonders guter Halt der Dichtelemente an dem Gestrick gewährleistet wird.

Nachfolgend wird die Erfindung anhand mehrerer Ausführungsbeispiele und in Verbindung mit den beigefügten Zeichnungen erläutert.

Dabei zeigt:
Figur 1 ein Ausführungsbeispiel des erfindungsgemäßen Prothesenliners, welcher aus einen über den Stumpf zu ziehenden Grundkörper sowie zumindest einen mit dem Grundkörper verbundenen ersten Gestrickabschnitt besteht, in einer Außenansicht,
Figur 2 einen Ausschnitt des erfindungsgemäßen Prothesenliners aus Figur 1 mit einem Grundkörper aus Silikon in einer Schnittdarstellung,
Figur 3 ein zweites Ausführungsbeispiel des erfindungsgemäßen Prothesenliners ebenfalls in einer Schnittdarstellung, jedoch mit einem Grundkörper gebildet aus einem Abstandsgestrick,
Figur 4 ein drittes Ausführungsbeispiel des erfindungsgemäßen Prothesenliners mit einem Grundkörper gefertigt aus einem Abstandsgestrick, dessen Wandstärke in Längsrichtung variiert,
Figur 5 ein viertes Ausführungsbeispiel des erfindungsgemäßen Prothesenliners mit ringförmigen Dichtelementen, gefertigt mit Silikonfäden,
Figur 6 ein fünftes Ausführungsbeispiel des erfindungsgemäßen Prothesenliners ebenfalls mit ringförmigen Dichtelementen, wobei diese nur teilweise bzw. abschnittweise aus Silikonfäden gefertigt sind,
Figur 7 ein Ausführungsbeispiel einer Prothesenanordnung bestehend aus einem Prothesenliner gemäß Figur 1 sowie einen ein Ventil aufweisenden Prothesenschaft;

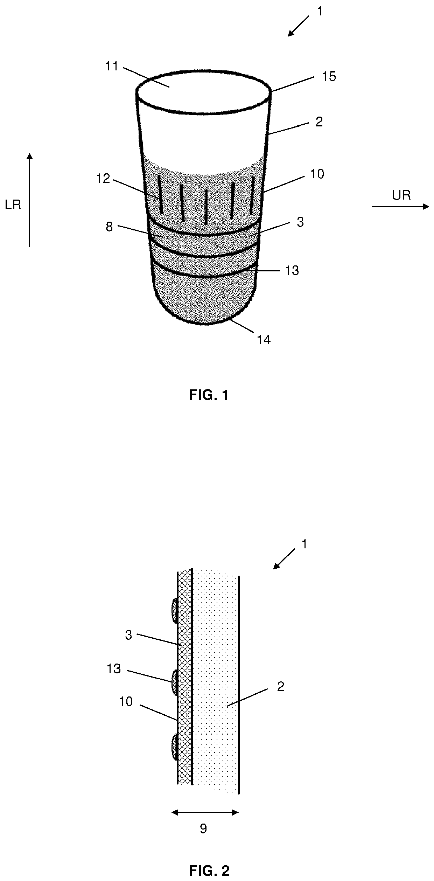

In Figur 1 ist ein erstes Ausführungsbeispiel des erfindungsgemäßen Prothesenliners 1, welcher aus einen über den Stumpf zu ziehenden hohlen Grundkörper 2 sowie zumindest einen mit dem Grundkörper 2 verbundenen ersten Gestrickabschnitt 3 besteht, gezeigt, nämlich in einer Außenansicht. Des Prothesenliner 1 besteht hierbei bevorzugt aus einem Grundkörper 2, gefertigt aus einem gegossenen Vollsilikon. Dieser bildete somit einen hohlen Grundkörper 2 mit einer Innenseite 11 und einer Außenseite 10 aus. An der Außenseite 10 ist der Gestrickabschnitt 3, insbesondere zum Ausgleich von Volumenschwankungen des Gliedmaßenstumpfes, bevorzugt unlösbar mit dem Silikonkörper 2 verbunden. Dieser Gestrickabschnitt 3, gefertigt vorzugswese aus einem Flach- oder Rundgestrick, insbesondere einem Recht-Rechts-, Rechts-Links oder Schlauchgesrick, erstreckt sich hierbei von einem unteren geschlossenen Ende 14 in Richtung eines oberen offenen Ende 15 des Prothesenliners 1 und deckt dabei ca. zweidrittel der Außenseite 10 des Prothesenliners 1 ab. In den Gestrickabschnitt 3 sind hierbei bevorzugt mehrere voneinander beabstandete Haftelemente 12 sowie Dichtelement 13 eingearbeitet. Die Haftelemente 12 erstrecken sich hierbei in Längsrichtung LR des Prothesenliners 1, um ein Verdrehen des Prothesenliners 1 in einem Prothesenschaft möglichst zu vermeiden. Die Dichtelemente 13 erstrecken sich in Umfangsrichtung UR, um den kompletten Prothesenliner 1 an mehreren unterschiedlichen Höhen mit dem Ziel, ein Vakuum zwischen dem Prothesenliner 1 und einem Prothesenschaft im Bereich des unteren Ende 14 des Prothesenliner 1 zu gewährleisten. Die Dicht- und Haftelemente 12, 13 werden erfindungsgemäß dadurch gebildet, dass in den ersten Gestrickabschnitt 3 zumindest abschnittsweise ein Haftfaden eingestrickt ist, insbesondere derart, dass dieser an der Oberseite des Gesrickabschnitts 3 auftritt, also zum Hervorschein kommt, und somit vorzugsweise adhäsiv mit dem Gliedmaßenstumpf und/ oder dem Prothesenschaft zusammenwirkt. Der Haftfaden kann hierbei auch im kompletten Gestrick verstrickt sein, also im Gestrickinneren, und nur Lokal an der Oberseite des Gestrickabschnitts 3 auftreten bzw. hervortreten. Die mehreren Haftelemente 12 weisen jeweils eine Ausdehnung in Gestrickumfangsrichtung zwischen 0,1 und 1 cm, maximal 2 cm, auf. In Gestricklängsrichtung können diese beliebigbar ausgebildet sein, vorzugsweise größer als 3 cm. Die Dichtelemente 13 weisen eine Ausdehnung in Gestricklängsrichtung vorzugsweise zwischen 0,1 und 1 cm, maximal 2 cm, auf.

Bevorzugt weist der erste Gestrickabschnitt 3 zumindest einen Kompressionsbereich 8 auf, um einen besonders guten Halt des Prothesenliner 1 an einem Gliedmaßenstumpf zu gewährleisten, wobei die Kompressionsstärke in dem Bereich zwischen 10 und 50 mmHg beträgt.

In Figur 2 ist ein Ausschnitt des erfindungsgemäßen Prothesenliners aus Figur 1 mit einem gegossenen Grundkörper 2 aus Silikon und einem daran bevorzugt unlösbar befestigten Gestrickabschnitt 3 in einer Schnittdarstellung gezeigt. Hierbei weist der Grundkörper 2 eine erste Wandstärke 9 auf. Die Innenseite des Grundkörpers 2 bildet die Innenseite des Prothesenliners 1. An der Außenseite des Grundkörpers 2 ist wiederum der Gestrickabschnitt 3 angeordnet, welcher die Außenseite 10 des Prothesenliners 1 bildet. In den Gestrickabschnitt 3 ist zur Bildung der radial umlaufenden Dichtelemente 13, wie zuvor beschrieben, ein Haftfaden 4 zumindest abschnittsweise eingestrickt. Wie in Figur 2 deutlich zu sehen ist, ist der Haftfaden 4 zur Bildung der Dichtelemente 13 derart in den ersten Gestrickabschnitt 3 eingestrickt, dass dieser an der Außenseite 10 des Prothesenliners 1 zumindest abschnittsweise hervortritt, also über das Gestrick hinausragt. Der Faden 4 ist hierbei bevorzugt auf einen Grundfaden 5 aufplattiert, oder alleine mit einer zum Grundstrickfaden unterschiedlichen Maschengröße gestickt.

Der Gestrickabschnitt 3 ist bevorzugt als Rundgestrick ausgebildet. Bei Ausbildung des Gestrickabschnitts 3 als Flachgestrick ist dieses vorzugsweise nahtlos gestrickt. Alternativ weist dieses eine seitlich verlaufende Naht auf. Dieses verschließt heribei bevorzugt auch das untere Ende des Gestrickabschnitts 3.

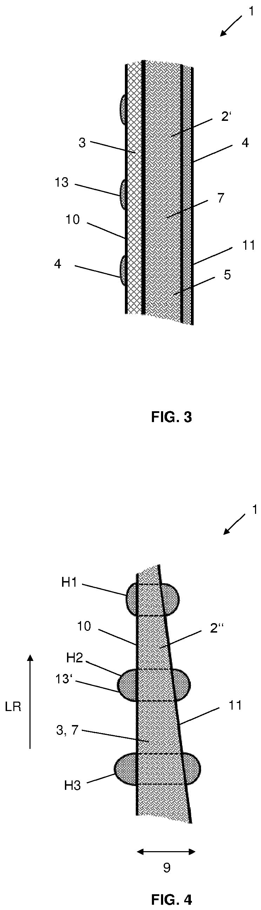

Figur 3 zeigt nun ein zweites Ausführungsbeispiel des erfindungsgemäßen Prothesenliners 1, ebenfalls in einer Schnittdarstellung, jedoch mit einem Grundkörper 2' gebildet aus einem zweiten Gestrickabschnitt 7, nämlich aus einem Abstandsgestrick. Anstelle des aus Silikon gegossenen Grundkörpers 2, wie in Figur 1 und 2 gezeigt, besteht nun der gesamte Prothesenliner 1 aus einem Gestrick, welches einen ersten und zweiten Gestrickabschnitt 3, 7 aufweist. Hierbei bildet der erste Gestrickabschnitt 3 die Außenseite 10 des Prothesenliners 1. In diesen ist, wie zuvor geschrieben, zur Bildung der radial umlaufenden Dichtelemente 13 ein Haftfaden 4 zumindest abschnittsweise eingestrickt. In den zweiten Gestrickabschnitt 7, vorzugsweise gebildet durch ein Abstandsgestrick, ist gemäß diesem Ausführungsbeispiel ebenfalls ein Haftfaden 4 eingestrickt und zwar derart, dass dieser die gesamte Innenseite 11 des Prothesenliners 1 bedeckt, so dass dieser vorzugsweise adhäsiv mit dem Gliedmaßenstumpf zusammenwirkt. Der Haftfaden 4 ist hierbei ebenfalls bevorzugt auf einen Grundfaden des zweiten Getrickabschnitts 7 aufplattiert, oder alleine mit einer zum Grundstrickfaden 5 unterschiedlichen Maschengröße gestickt. Der erste und zweite Gestrickabschnitt 3, 7 können natürlich durch ein einziges Gestrickteil gefertigt sein. Die Bezugnahme auf mehrere Abschnitte dient lediglich zur besseren Veranschaulichung.

In Figur 4 ist nun ein drittes Ausführungsbeispiel des erfindungsgemäßen Prothesenliners 1 mit einem Grundkörper 2" gefertigt aus einem Abstandsgestrick gezeigt, dessen Wandstärke 9 in Längsrichtung LR variiert. Der erste und zweite Gestrickabschnitt 3, 7 bilden ein Gestrickteil mit einer Wandstärke 9. Diese nimmt in Gestricklängsrichtung LR ab. Zusätzlich oder alternativ kann die Wandstärke 9 des Grundkörpers 2", insbesondere die des Abstandgestricks, in Umfangsrichtung variieren abhängig von der Geometrie des Gliemaßenstumpfes. Daher ist die Dicke des Prothesenliners 1 individuell, also punktgenau bzw. maschengenau, herstellbar.

Wie ferner zu sehen ist, sind an der Außenseite 10 und an der Innenseite 11 des Grundkörpers 2" mehrere Dichtelemente 13' angeordnet. Zur Bildung dieser ist der Haftfaden 4 derart in den ersten und zweiten Gestrickabschnitt 3, 7 eingestrickt, dass sich dieser durch das Gestrick erstreckt und an der Außen- und Innenseite 10, 11 des Prothesenliners 1 zumindest abschnittsweise hervortritt. Die gegenüberliegenden Dichtelemente 13' sind somit miteinander verbunden. Vorzugsweise ist der Haftfaden 4 derart eingestrickt, dass die Höhe der Dichtelemente 13' variiert. Im konkreten Ausführungsbeispiel weisen die drei Dichtelemente 13' an der Innenseite 10 des Grundkörpers 2" die selbe Höhe auf. An der Außenseite 11 unterscheidet sich jedoch die Höhe der Dichtelemente 13'. Hier weist das oberste Element eine Höhe H1, das mittlere Element eine Höhe H2, sowie das unterste Element eine Höhe H3 auf, wobei gilt H1 < H2 < H3. De Höhe der Dichtelemente 13' beträgt vorzugsweise zwischen 2 - 8 mm. Bei dem Haftfaden handelt es sich, wie bei den zuvor gezeigten Ausführungsbeispielen, vorzugsweise um einen Silikon-, Elastan- oder Kautschukfaden.

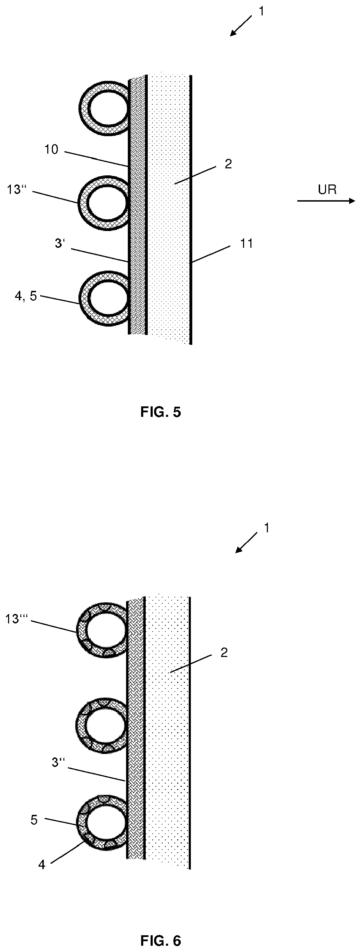

Figur 5 zeigt schließlich ein viertes Ausführungsbeispiel des erfindungsgemäßen Prothesenliners 1 mit ringförmigen Dichtelementen 13", gefertigt aus Grundstrick- und eingestrickten Haftfäden 4, 5. Der Grundkörper 2 besteht hier ebenfalls, wie in Figur 1 und 2 gezeigt, aus einem gegossenen Vollsilikon, welches die Innenseite 11 des Prothesenliners 1 bildet. Dieser Grundkörper 2 kann des Weiteren einen dritten Gestrickabschnitt in Form eines textilen Verstärkungsmaterials aufweisen. Zur Bildung der mehreren Dichtelemente 13" ist nun der erste Gestrickabschnitt 3' derart ausgebildet, dass dieser an der Außenseite 10 des Prothesenliners 1 zumindest abschnittsweise hervortritt. Der Gestrickabschnitt 3' bildet somit bevorzugt lokale Dichtelemente 13" mit ringförmigen Querschnittsformen aus. Dies geschieht dadurch, dass der erste Gestrickabschnitt 3' eingestrickte Wellen aufweist, die durch eine Anhäufung von linken Maschen des Grundstrickfadens 5 ausgebildet werden. Hierdurch entstehen die gezeigten schlauchförmigen Wülste, welche sich um den Prothesenliner 1 in Umfangsrichtung UR herum erstrecken. Dadurch sind besonders Hohe Dichtelemente 13" herstellbar. In diesen erzeugten Wellen ist bevorzugt der Haftfaden 4 vollflächig eingestrickt, insbesondere vollflächig auf den Grundstrickfaden 5 aufplattiert, um adhäsiv mit dem Prothesenschaft zusammenwirken.

Figur 6 zeigt nun ein weiteres Ausführungsbeispiel des Prothesenliners 1 mit einem Grundkörper 2, an dem ein erster Gestrickabschnitt 3" mit ringförmigen Dichtelementen 13'" angeordnet ist, wobei diese aus einem oder mehreren Grundstrickfäden 5 und nur teilweise bzw. abschnittweise aus Haftfäden 4 gefertigt sind. Hier ist der Haftfaden 4 nur partiell im Bereich der Wellen eingestrickt bzw. aufplattiert und bildet dadurch nur lokale Dichtbereiche auf den Dichtelementen 13'" aus. Es ist übrigens festzuhalten, dass auch die Haftelemente gemäß Figur 1 auch gemäß der sämtlichen dargestellten Ausführungsformen der Dichtelemente ausgeführt werden können.

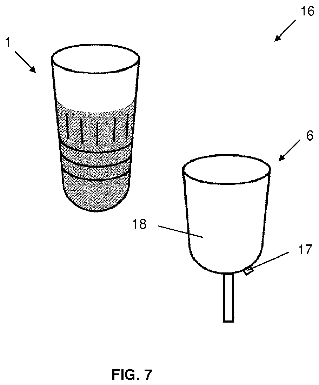

In Figur 7 ist nun eine Prothesenanordnung 16 gezeigt, welche aus einem Prothesenliner 1 gemäß der Figur 1 sowie einen ein Ventil 17 aufweisenden Prothesenschaft 6 besteht. Der Prothesenschaft 6 weist hierbei eine hohle Schale 18 auf, in der der Gliedmaßenstumpf mit dem übergezogenen Prothesenliner 1 aufnembar ist.

Die Erfindung ist nicht auf die beschriebenen Ausführungsbeispiele beschränkt. Es sind alle Merkmale aller beschriebenen und dargestellten Ausführungsbeispiele miteinander kombinierbar.

## Patentansprüche

1. Prothesenliner (1) zum Überziehen über einen Gliedmaßenstumpf zur Fixierung eines Prothesenschafts (6), bestehend aus einen über den Stumpf zu ziehenden Grundkörper (2, 2', 2") sowie zumindest einen mit dem Grundkörper (2, 2', 2") verbundenen ersten Gestrickabschnitt (3, 3', 3"), **dadurch gekennzeichnet, dass** in den zumindest einen ersten Gestrickabschnitt (3, 3', 3") zumindest abschnittsweise ein Haftfaden (4) eingestrickt ist, der vorzugsweise adhäsiv mit dem Gliedmaßenstumpf und/ oder dem Prothesenschaft (6) zusammenwirkt.

2. Prothesenliner (1) nach Anspruch 1, **dadurch gekennzeichnet, dass** der zumindest eine erste Gestrickabschnitt (3, 3', 3") lösbar mit dem Grundkörper (2, 2', 2") verbunden ist.

3. Prothesenliner (1) nach Anspruch 1, **dadurch gekennzeichnet, dass** der zumindest eine erste Gestrickabschnitt (3, 3', 3") unlösbar mit dem Grundkörper (2, 2', 2") verbunden ist.

4. Prothesenliner (1) nach Anspruch 3, **dadurch gekennzeichnet, dass** der Grundkörper (2', 2") durch einen zweiten Gestrickabschnitt (7), insbesondere einem Abstandsgestrick, gebildet ist.

5. Prothesenliner (1) nach einem der vorherigen Ansprüche, **dadurch gekennzeichnet, dass** der erste und/ oder zweite Gestrickabschnitt (3, 3', 3"; 7) zumindest einen Kompressionsbereich (8) aufweist, wobei die Kompressionsstärke in dem Bereich zwischen 10 und 50 mmHg beträgt.

6. Prothesenliner (1) nach einem der vorherigen Ansprüche, **dadurch gekennzeichnet, dass** die Wandstärke (9) des Grundkörpers (2"), insbesondere die des Abstandgestricks, in Umfangsrichtung (UR) und/ oder Längsrichtung (LR) variiert.

7. Prothesenliner (1) nach einem der vorherigen Ansprüche, **dadurch gekennzeichnet, dass** der erste und/ oder zweite Gestrickabschnitt (3', 3") zur Bildung eines oder mehrerer Haft- und/ oder Dichtelemente (13", 13'") derart ausgebildet ist, dass dieser an der Außen- und/ oder Innenseite (10, 11) des Prothesenliners (1) zumindest abschnittsweise hervortritt.

8. Prothesenliner (1) nach Anspruch 7, **dadurch gekennzeichnet, dass** das eine oder die mehreren gebildeten Haft- und/ oder Dichtelemente (13", 13'") eine ringförmige Querschnittsform aufweisen.

9. Prothesenliner (1) nach einem der vorherigen Ansprüche, **dadurch gekennzeichnet, dass** der Haftfaden (4) zur Bildung eines oder mehrerer Haft- und/ oder Dichtelemente (13, 13') derart in den ersten und/ oder zweiten Gestrickabschnitt (3, 7) eingestrickt ist, dass dieser an der Außen- und/ oder Innenseite (10, 11) des Prothesenliners (1) zumindest abschnittsweise hervortritt.

10. Prothesenliner (1) nach einem der Ansprüche 7 bis 9, **dadurch gekennzeichnet, dass** das eine oder die mehreren Haft- und/ oder Dichtelemente (12; 13, 13', 13", 13'") jeweils eine Ausdehnung in Gestricklängs- und/ oder Umfangsrichtung (LR, UR) zwischen 0,1 und 1 cm, maximal 2 cm, aufweisen.

11. Prothesenliner (1) nach einem der Ansprüche 7 oder 9, **dadurch gekennzeichnet, dass** die mehreren Haft- und/ oder Dichtelemente (13') unterschiedlich hoch sind.

12. Prothesenliner (1) nach einem der Ansprüche 7 oder 10, **dadurch gekennzeichnet, dass** die Höhe (H1) eines ersten Haft- und/ oder Dichtelements (13') zwischen 4 - 8 mm, insbesondere 6 mm, und die Höhe (H2, H3) eines zweiten Haft- und/ oder Dichtelements (13') zwischen 2 - 5 mm, insbesondere 4 mm, beträgt.

13. Prothesenliner (1) nach einem der Ansprüche 7 oder 12, **dadurch gekennzeichnet, dass** die Höhe (H1, H2, H3) eines, mehrerer oder aller Haft- und/ oder Dichtelemente (13') variiert.

14. Prothesenliner (1) nach einem der vorherigen Ansprüche, **dadurch gekennzeichnet, dass** der Haftfaden (4) ein Silikon-, Elastan- oder Kautschukfaden ist.

15. Prothesenanordnung (16) bestehend aus einem Prothesenliner (1) nach einem der vorhergehenden Ansprüchen sowie einen ein Ventil (17) aufweisenden Prothesenschaft (6).
